# EUROPÄISCHE PATENTSCHRIFT

(11) **EP 3 914 315 B1**
(45) Veröffentlichungstag und Bekanntmachung des Hinweises auf die Patenterteilung: **25.12.2024**
(21) Anmeldenummer: 20702976.0
(22) Anmeldetag: 24.01.2020
(51) Int. Cl.: A61M 1/16

(54) **VORRICHTUNG ZUR EXTRAKORPORALEN BLUTBEHANDLUNG**
DEVICE FOR AN EXTRACORPOREAL BLOOD TREATMENT
DISPOSITIF DE TRAITEMENT EXTRACORPOREL DU SANG

(30) Priorität: 24.01.2019 DE 102019101774
(43) Veröffentlichungstag der Anmeldung: 01.12.2021
(73) Patentinhaber: Fresenius Medical Care Deutschland GmbH, 61352 Bad Homburg (DE)
(72) Erfinder: SPICKERMANN, Reiner, 97535 Burghausen (DE); MÜLLER, Carsten, 97502 Euerbach (DE); KOPPERSCHMIDT, Pascal, 97456 Dittelbrunn (DE); MAIERHOFER, Andreas, 97422 Schweinfurt (DE); GAGEL, Alfred, 96123 Litzendorf (DE); WIESEN, Gerhard, 61352 Bad Homburg (DE); SMYSLOVA, Liubov, 61352 Bad Homburg (DE)
(74) Vertreter: Herrmann, Uwe
(86) Internationale Anmeldenummer: PCT/EP2020/051762
(87) Internationale Veröffentlichungsnummer: WO 2020/152333

(56) Entgegenhaltungen:
- EP-A1- 0 613 688
- US-A- 4 722 798
- US-A1- 2013 327 713

## Beschreibung

Die vorliegende Erfindung betrifft eine Vorrichtung zur extrakorporalen Blutbehandlung mit einem extrakorporalen Blutkreislauf, in dem ein Dialysator angeordnet ist, sowie mit einem Dialysatkreislauf, wobei der Blutkreislauf mit einer ersten Kammer und der Dialysatkreislauf mit einer zweiten Kammer des Dialysators in Fluidverbindung stehen und wobei die beiden Kammern durch eine semipermeable Membran voneinander getrennt sind, wobei in dem Dialysatkreislauf eine Dialysatpumpe zur Förderung der Dialyelösung vorhanden ist.

Derartige Vorrichtungen dienen zur Entfernung harnpflichtiger Substanzen aus dem Blut des Patienten, die über die Membran des Dialysators in die Dialyselösung übertreten und auf diese Weise aus dem Blut des Patienten entfernt werden.

Eine Vorrichtung mit den Merkmalen des Oberbegriffs des Anspruchs 1 ist aus der EP 0 942 759 B1 bekannt. Bei der aus dieser Druckschrift bekannten Vorrichtung ist vorgesehen, während der Behandlung die tatsächliche Dialyseeffizienz (K/V) an einen für den Patienten noch erträglichen Wert für die maximale Dialyseeffizienz (K/V)ₘₐₓ anzunähern, d.h. die Behandlung von Beginn an mit einer möglichst hohen Effizienz zu betreiben, um die Behandlungsdauer so kurz wie möglich zu halten. Die Behandlung wird beendet, wenn die vorgeschriebene Dialysedosis (Kt/V) erreicht ist, wobei t die Behandlungsdauer, K die Clearance und V das Verteilungsvolumen des Patienten ist.

Bei der Dialyse können aufgrund des Übergangs von Elektrolyten oder sonstigen Substanzen, wie z.B. von Kalium-Ionen oder Harnstoff vom Blut in die Dialyselösung Komplikationen auftreten, die zu Beschwerden, wie Kopfschmerzen, Erbrechen etc. führen können, was auch als Dysäquilibrium-Syndrom bekannt ist.

US 2013/327713 A1 betrifft eine Vorrichtung zur extrakorporalen Blutbehandlung mit einem extrakorporalen Blutkreislauf, der eine erste Kammer eines Dialysators oder Filters enthält, die durch eine semipermeable Membran in eine erste und eine zweite Kammer unterteilt ist, und mit einem Dialysierflüssigkeitssystem, das die zweite Kammer des Dialysators oder Filters enthält.

EP 0 613 688 A1 offenbart eine Vorrichtung zur Bereitung von bicarbonathaltigen Dialysierflüssigkeiten für die Hämodialyse.

US 4 722 798 A betrifft ein Hämodialyseverfahren, bei dem ein Dialysator verwendet wird, der ein Blutstromkompartiment aufweist, das von einem Dialysatstromkompartiment durch eine semipermeable Membran getrennt ist.

Der vorliegenden Erfindung liegt die Aufgabe zugrunde, eine Vorrichtung der eingangs genannten Art dahingehend weiterzubilden, dass die Wahrscheinlichkeit für das Auftreten derartiger Komplikationen und/oder die Schwere der Komplikationen gegenüber bekannten Geräten verringert ist.

Diese Aufgabe wird durch eine Vorrichtung mit den Merkmalen des Anspruchs 1 gelöst.

Danach ist vorgesehen, dass die Vorrichtung eine Steuereinheit aufweist, die ausgebildet ist, die Vorrichtung in einer ersten Phase und einer der ersten Phase folgenden zweiten Phase zu betreiben, wobei die Dialysatpumpe in der ersten Phase mit einer geringeren Flussrate betrieben wird als in der zweiten Phase und wobei die Dialysatpumpe in der ersten Phase eine bzgl. wenigstens einer Komponente höher konzentrierte Dialyselösung fördert als in der zweiten Phase.

Die zweite Phase schließt sich vorzugsweise unmittelbar an die erste Phase an. Von der Erfindung ist jedoch der Fall umfasst, dass die zweite Phase zeitlich beabstandet zum Ende der ersten Phase beginnt.

Der vorliegenden Erfindung liegt somit der Gedanke zugrunde, den Dialysatfluss zu Beginn der Behandlung geringer zu wählen als zu einem späteren Zeitpunkt der Behandlung bzw. zu Beginn der Behandlung eine bzgl. einer oder mehrerer Substanzen höher konzentrierte Dialyselösung zu verwenden als zu einem späteren Zeitpunkt der Behandlung. Vorzugsweise handelt es sich bei dieser oder diesen Substanzen um solche, die auch im Blut vorkommen, wie z.B. Natrium-Ionen etc.

Durch beide Maßnahmen wird erreicht, dass der Entzug von harnpflichten Substanzen aus dem Blut zu Beginn der Behandlung vergleichsweise langsam erfolgt, was zu einer gesteigerten Verträglichkeit der Behandlung beim Patienten führt und die Wahrscheinlichkeit für das Auftreten des Dysäquilibrium-Syndroms bzw. die Schwere der Symptomatik erheblich verringert. Die verschriebene Flussrate bzw. Konzentration der Dialyselösung wird somit nicht von Beginn an erreicht bzw. eingestellt, sondern erst zu einem späteren Zeitpunkt der Behandlung, z.B. nach 30 min.

Wird in der ersten Phase ein vergleichsweise geringer Dialysatfluss eingestellt, erfolgt ein entsprechend langsamer diffusiver Übergang von Substanzen aus dem Blut in die Dialyselösung. Entsprechendes gilt, wenn die Konzentration einer auch im Blut vorkommenden und darin abzureichernden Komponente in der Dialyselösung zunächst hoch eingestellt wird, so dass der Konzentrationsgradient zwischen Blut und Dialyselösung gering ist, was ebenfalls zu einer zunächst geringen Diffusionsrate aus dem Blut führt.

Die Steuereinheit kann derart ausgebildet sein, dass die Flussrate und/oder Konzentration in der ersten Phase und/oder in der zweiten Phase konstant ist oder sich verändert, wobei die Veränderung vorzugsweise linear, exponentiell oder stufenweise erfolgt. Grundsätzlich ist jede beliebige Profilierung der Flussrate und/oder der Konzentration von der Erfindung umfasst.

Denkbar ist es, dass nur in der ersten Phase eine fest vorgegebene oder von einem oder mehreren Parametern abhängige Profilierung der Flussrate und/oder der Konzentration wenigstens einer Substanz der Dialyselösung erfolgt und dass in der zweiten Phase eine Einstellung der Flussrate und/oder der Konzentration wenigstens einer Substanz der Dialyselösung nach anderen Kriterien erfolgt als in der ersten Phase, beispielsweise in Abhängigkeit von Messwerten, wie z.B. der gemessenen Clearance.

Die Steuereinheit kann derart ausgeführt sein, dass die Änderung der Flussrate der Dialyselösung und/oder der Konzentration der fraglichen Komponente(n) in der Dialyselösung nur in der ersten Phase, nur in der zweiten Phase oder sowohl in der ersten als auch in der zweiten Phase erfolgt. Denkbar ist, dass das Dialysegerät in der ersten und/oder zweiten Phase mit einer konstanten Flussrate und/oder Konzentration bzgl. der Dialyselösung betrieben wird.

Denkbar ist es, dass die Steuereinheit derart ausgebildet ist, dass in der zweiten Phase keine Änderung der Flussrate der Dialyselösung und/oder keine Änderung der Konzentration der Dialyselösung erfolgt.

Die Steuereinheit kann derart ausgebildet sein, dass sich die erste Phase über eine Zeitspanne von 15 min. bis 60 min. erstreckt, vorzugsweise über eine Zeitspanne von 20 min. bis 40 min. und besonders bevorzugt über einen Zeitraum von 30 min.

Bei den vorgenannten Werten handelt es sich selbstverständlich um Beispiele, die die Erfindung nicht beschränken.

Die Steuereinheit kann derart ausgebildet sein, dass vor der ersten Phase eine Konditionierungsphase z.B. für eine Dauer von 5 min. bis 10 min. erfolgt, in der keine Dialyse, sondern nur eine Hämofiltration stattfindet. In dieser Phase, die den Beginn der Behandlung darstellen kann, besteht somit nur eine konvektive Clearance aufgrund eines Druckgefälles über die Membran, aber keine diffusive Clearance.

An diese Konditionierungsphase schließt sich dann unmittelbar oder zeitlich beabstandet die erste Phase der Behandlung an. Eine solche Konditionsphase ist z.B. aus der DE 10 2016 008 755 A1 bekannt.

Die Dauer der ersten Phase kann für alle Patienten konstant sein oder von einem oder mehreren Behandlungs- und/oder Patientenparametern abhängig sein, wie z.B. von dem Körpergewicht und/oder von dem Verteilungsvolumen des Patienten und/oder von der Stoffkonzentration im Blut, wie z.B. von der prädialytischen Harnstoffkonzentration. Die Dauer der zweiten Phase richtet sich vorzugsweise danach, wann die vorgeschriebene Dialysedosis erreicht ist.

Die Steuereinheit kann ausgeführt sein, dass in der zweiten Phase die Flussrate und/oder die Konzentration in Abhängigkeit der während der Behandlung ermittelten Clearance oder erreichten Dialysedosis eingestellt wird. Denkbar ist somit ein onlineclearance-monitoring, d.h. eine in Echtzeit erfolgende Clearance-Messung und in Abhängigkeit davon die Einstellung der Flussrate und/oder der Konzentration der Dialyselösung.

Auch ist es denkbar, vor oder zu Beginn der Behandlung ein bestimmtes Profil für die Einstellung der Flussrate und/oder der Konzentration der Dialyselösung für die erste und/oder für die zweite Phase einzustellen, das sodann von der Steuereinheit und unabhängig von etwaigen Messwerten durchlaufen wird.

Der Übergang von der ersten in die zweite Phase kann hinsichtlich der Konzentration und/oder hinsichtlich des Dialysatflusses kontinuierlich oder stufenweise erfolgen.

Denkbar ist es beispielsweise, in der ersten Phase eine bestimmte erste Flussrate und/oder Konzentration der Dialyselösung und in der zweiten Phase oder jedenfalls zu deren Beginn oder dauerhaft eine zweite Flussrate und/oder Konzentration der Dialyselösung einzustellen, so dass sich ein stufenartiger Übergang ergibt.

Von der Erfindung ist hinsichtlich der Flussrate und/oder Konzentration der Dialyselösung jedoch auch ein kontinuierlicher Übergang von der ersten zu der zweiten Phase umfasst.

Die Steuereinheit kann ausgebildet sein, die Vorrichtung als Hämodialysegerät oder als Hämodiafiltrationsgerät zu betreiben. In anderen Worten kann es sich bei dem Gerät um ein Hämodialysegerät oder um ein Hämodiafiltationsgerät handeln. Denkbar und von der Erfindung umfasst ist auch der Fall, dass das Gerät zeitweise als reines Hämofiltrationsgerät betrieben wird, d.h. ohne dass im Dialysator Dialyselösung vorliegt.

Ebenfalls ist es denkbar, dass das Gerät über eine oder mehrere Leitungen für ein Substitutionsfluid verfügt, das nur stromaufwärts, nur stromabwärts oder sowohl stromaufwärts als auch stromabwärts des Dialysators dem Blut zugegeben wird. Dabei kann die Steuereinheit ausgeführt sein, den Fluss der Substitutionslösung in der ersten Phase geringer einzustellen als in der zweiten Phase. Denkbar ist es beispielsweise, den Fluss an Substitutionslösung von dem Wert Null zu Beginn der Behandlung auf den verschriebenen Wert steigen zu lassen und/oder die Substitutionsrate von der Flussrate der Dialyselösung abhängig zu gestalten.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

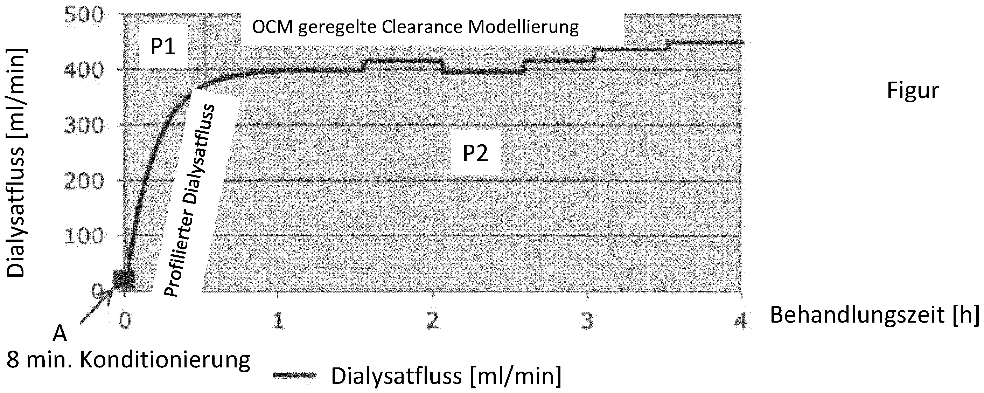

Weitere Einzelheiten und Vorteile der Erfindung ergeben sich aus einem in der Zeichnung dargestellten Ausführungsbeispiel.

Die einzige Figur zeigt den Verlauf der Flussrate der Dialyselösung über die Zeit bei einer Vorrichtung gemäß der Erfindung.

Auf der Ordinate ist die Flussrate der durch den Dialysator strömenden Dialyselösung und auf der Abszisse die Zeit dargestellt.

Wie dies aus der Figur hervorgeht, erfolgt nach einer Konditionierungsphase (Punkt A), in der kein diffusiver, sondern nur ein konvektiver Stofftransport vom Blut über die Membran in die Dialyselösung erfolgt, in einer ersten Phase P1 ein Anstieg der Flussrate der Dialyselösung durch den Dialysator, wobei der Anstieg mit zunehmender Zeit in der ersten Phase kleiner wird.

Die vertikale Linie in der Figur kennzeichnet die Grenze zwischen der ersten und der zweiten Phase. In der zweiten Phase P2 ist die Flussrate der Dialyselösung höher als in der ersten Phase und weitgehend konstant.

Der Übergang des Verlaufs der Flussrate von der ersten zur zweiten Phase erfolgt wir aus der Figur ersichtlich stetig und ohne Stufe.

In der ersten Phase P1 ist der Verlauf der Flussrate profiliert, wobei das Profil fest vorgegeben sein kann oder von einem oder mehreren Parametern abhängen kann, wie z.B. vom Zustand des Patienten, vom Körpergewicht des Patienten, von dessen Verteilungsvolumen etc.

In der zweiten Phase P2 erfolgt die Einstellung der Flussrate der Dialyselösung in Abhängigkeit von der in der zweiten Phase gemessenen Clearance K (OCM controlled clearance modelling) und/oder in Abhängigkeit von der verschriebenen Behandlungszeit, in der eine bestimmte Dialysedosis erreicht werden muss oder gemäß einen verschrieben Sollwert oder Sollwertprofil.

Wie dies aus der Figur hervorgeht, wird zu Beginn der Behandlung eine schnelle Entfernung von Salzen, Harnstoff etc. wegen des entstehenden Disäquilibriums mit seinen damit verbundenen Folgen gezielt verhindert, indem ein vergleichsweise geringer Dialysatfluss eingestellt wird. Die eigentlich verschriebene Flussrate der Dialyselösung wird also nicht durch möglichst schnelles Hochfahren der Dialysatpumpe erreicht, sondern durch langsames Steigern der Flussrate bewusst zeitlich verzögert erreicht.

Das Erreichen der Flussrate in der zweiten Phase kann stufenweise oder kontinuierlich erfolgen, wie dies aus der Figur hervorgeht.

Ein zu Beginn der Behandlung langsamerer Entzug harnpflichtiger Substanzen gegenüber der späteren Behandlung kann auch dadurch erreicht werden, dass zu Beginn der Behandlung eine andere Dialyselösung verwendet wird, als zu einem späteren Zeitpunkt der Behandlung. Auch so lässt sich eine zunächst geringe und dann höhere Verringerung der Konzentration der fraglichen Stoffe im Blut erreichen. Bei dieser Vorgehensweise ist es denkbar, dass verschiedene Dialyselösungen verwendet werden, die in verschiedenen Beuteln etc. gelagert sind oder dass in ein und demselben Reservoir der Dialyselösung die Konzentration eines oder mehrere Inhaltsstoffe linear oder stufenweise geändert wird.

## Patentansprüche

1. Vorrichtung zur extrakorporalen Blutbehandlung mit einem extrakorporalen Blutkreislauf, in dem ein Dialysator angeordnet ist, sowie mit einem Dialysatkreislauf, wobei der Blutkreislauf mit einer ersten Kammer und der Dialysatkreislauf mit einer zweiten Kammer des Dialysators in Fluidverbindung stehen und wobei die beiden Kammern durch eine semipermeable Membran voneinander getrennt sind, wobei in dem Dialysatkreislauf eine Dialysatpumpe zur Förderung der Dialyelösung vorhanden ist, **dadurch gekennzeichnet, dass** die Vorrichtung eine Steuereinheit aufweist, die ausgebildet ist, die Vorrichtung in einer ersten Phase und einer der ersten Phase folgenden zweiten Phase zu betreiben, wobei die Dialysatpumpe in der ersten Phase mit einer geringeren Flussrate betrieben wird als in der zweiten Phase und wobei die Dialysatpumpe in der ersten Phase eine bzgl. wenigstens einer Komponente höher konzentrierte Dialyselösung fördert als in der zweiten Phase.

2. Vorrichtung nach Anspruch 1, **dadurch gekennzeichnet, dass** die Steuereinheit derart ausgebildet ist, dass die Flussrate und/oder Konzentration in der ersten Phase und/oder in der zweiten Phase konstant ist oder sich verändert, wobei die Veränderung vorzugsweise linear, exponentiell oder stufenweise erfolgt.

3. Vorrichtung nach Anspruch 2, **dadurch gekennzeichnet, dass** die Steuereinheit derart ausgeführt ist, dass die Änderung der Flussrate und/oder der Konzentration nur in der ersten Phase oder sowohl in der ersten als auch in der zweiten Phase erfolgt.

4. Vorrichtung nach einem der vorhergehenden Ansprüche, **dadurch gekennzeichnet, dass** die Steuereinheit derart ausgebildet ist, dass in der zweiten Phase keine Änderung der Flussrate und/oder keine Änderung der Konzentration erfolgt.

5. Vorrichtung nach einem der vorhergehenden Ansprüche, **dadurch gekennzeichnet, dass** die Steuereinheit derart ausgebildet ist, dass sich die erste Phase über eine Zeitspanne von 15 min. bis 60 min. erstreckt, vorzugsweise über eine Zeitspanne von 20 min. bis 40 min. und besonders bevorzugt über einen Zeitraum von 30 min.

6. Vorrichtung nach einem der vorhergehenden Ansprüche, **dadurch gekennzeichnet, dass** die Steuereinheit derart ausgebildet ist, dass vor der ersten Phase eine Konditionierungsphase erfolgt, in der keine Dialyse, sondern nur eine Hämofiltration stattfindet.

7. Vorrichtung nach einem der vorhergehenden Ansprüche, **dadurch gekennzeichnet, dass** die Steuereinheit derart ausgebildet ist, dass die Dauer der ersten Phase von einem oder mehreren Behandlungs- und/oder Patientenparametern abhängig ist.

8. Vorrichtung nach Anspruch 7, **dadurch gekennzeichnet, dass** es sich bei den Patientenparametern um das Körpergewicht und/oder um das Verteilungsvolumen des Patienten und/oder um die Stoffkonzentration im Blut, wie z.B. die prädialytische Harnstoffkonzentration handelt.

9. Vorrichtung nach einem der vorhergehenden Ansprüchen, **dadurch gekennzeichnet, dass** die Steuereinheit ausgeführt ist, dass in der zweiten Phase die Flussrate und/oder die Konzentration in Abhängigkeit der während der Behandlung ermittelten Clearance oder erreichten Dialysedosis eingestellt wird.

10. Vorrichtung nach einem der vorhergehenden Ansprüche, **dadurch gekennzeichnet, dass** die Steuereinheit ausgeführt ist, dass der Übergang von der ersten in die zweite Phase hinsichtlich der Konzentration und/oder hinsichtlich des Dialysatflusses kontinuierlich oder stufenweise erfolgt.

11. Vorrichtung nach einem der vorhergehenden Ansprüche, **dadurch gekennzeichnet, dass** die Steuereinheit ausgebildet ist, die Vorrichtung als Hämodialysegerät oder als Hämodiafiltrationsgerät und ggf. zeitweise als Hämofiltrationsgerät zu betreiben.

## Claims

1. An apparatus for an extracorporeal blood treatment having an extracorporeal blood circuit in which a dialyzer is arranged and having a dialyzate circuit, wherein the blood circuit is in fluid communication with a first chamber and the dialyzate circuit is in fluid communication with a second chamber of the dialyzer, and wherein the two chambers are separated from one another by a semipermeable membrane, with a dialyzate pump for a conveying of the dialysis solution being present in the dialyzate circuit, **characterized in that** the apparatus has a control unit that is configured to operate the apparatus in a first phase and in a second phase following the first phase, wherein the dialyzate pump is operated with a smaller flow rate in the first phase than in the second phase and wherein the dialyzate pump conveys a dialysis solution in the first phase that is of a higher concentration with respect to at least one component than in the second phase.

2. An apparatus in accordance with claim 1, **characterized in that** the control unit is configured such that the flow rate and/or the concentration is constant or varies in the first phase and/or in the second phase, with the variation preferably taking place linearly, exponentially, or step-wise.

3. An apparatus in accordance with claim 2, **characterized in that** the control unit is designed such that the variation of the flow rate and/or of the concentration only takes place in the first phase or both in the first and second phases.

4. An apparatus in accordance with one of the preceding claims, **characterized in that** the control unit is configured such that no variation of the flow rate and/or no variation of the concentration takes place in the second phase.

5. An apparatus in accordance with one of the preceding claims, **characterized in that** the control unit is configured such that the first phase extends over a time span of 15 min. to 60 min., preferably over a time span of 20 min. to 40 min., and particularly preferably over a time period of 30 min.

6. An apparatus in accordance with one of the preceding claims, **characterized in that** the control unit is configured such that a conditioning phase in which no dialysis takes place, but rather only hemofiltration, takes place before the first phase.

7. An apparatus in accordance with one of the preceding claims, **characterized in that** the control unit is configured such that the duration of the first phase is dependent on one or more treatment parameters and/or patient parameters.

8. An apparatus in accordance with claim 7, **characterized in that** the patient parameters are the body weight and/or the distribution volume of the patient and/or the substance concentration in the blood such as the predialytic urea concentration.

9. An apparatus in accordance with one of the preceding claims, **characterized in that** the control unit is designed such that the flow rate and/or the concentration is/are set in the second phase in dependence on the clearance determined during the treatment or on the dialysis dosage reached during the treatment.

10. An apparatus in accordance with one of the preceding claims, **characterized in that** the control unit is designed such that the transition from the first phase into the second phase takes place continuously or step-wise with respect to the concentration and/or with respect to the dialyzate flow.

11. An apparatus in accordance with one of the preceding claims, **characterized in that** the control unit is configured to operate the apparatus as a hemodialysis machine or as a hemodiafiltration machine and optionally at times as a hemofiltration machine

## Revendications

1. Dispositif pour le traitement extracorporel du sang avec un circuit de sang extracorporel, dans lequel est agencé un dialyseur, ainsi qu'avec un circuit de dialysat, le circuit de sang étant en communication fluidique avec une première chambre et le circuit de dialysat avec une deuxième chambre du dialyseur et les deux chambres étant séparées l'une de l'autre par une membrane semi-perméable, une pompe à dialysat étant présente dans le circuit de dialysat pour le transport de la solution de dialyse, **caractérisé en ce que** le dispositif présente une unité de commande qui est configurée pour faire fonctionner le dispositif dans une première phase et dans une deuxième phase suivant la première phase, la pompe à dialysat fonctionnant dans la première phase avec un débit plus faible que dans la deuxième phase et la pompe à dialysat transportant dans la première phase une solution de dialysat plus concentrée en ce qui concerne au moins un composant que dans la deuxième phase.

2. Dispositif selon la revendication 1, **caractérisé en ce que** l'unité de commande est configurée de telle sorte que le débit et/ou la concentration dans la première phase et/ou dans la deuxième phase est constant ou varie, la variation étant de préférence linéaire, exponentielle ou par étapes.

3. Dispositif selon la revendication 2, **caractérisé en ce que** l'unité de commande est conçue de telle sorte que la modification du débit et/ou de la concentration se produit uniquement dans la première phase ou à la fois dans la première et dans la deuxième phase.

4. Dispositif selon l'une quelconque des revendications précédentes, **caractérisé en ce que** l'unité de commande est configurée de telle sorte qu'aucune modification du débit et/ou aucune modification de la concentration ne se produit dans la deuxième phase.

5. Dispositif selon l'une quelconque des revendications précédentes, **caractérisé en ce que** l'unité de commande est configurée de telle sorte que la première phase s'étend sur une période de temps de 15 minutes à 60 minutes, de préférence sur une période de temps de 20 minutes à 40 minutes et de manière particulièrement préférée sur une période de temps de 30 minutes.

6. Dispositif selon l'une quelconque des revendications précédentes, **caractérisé en ce que** l'unité de commande est configurée de telle sorte que la première phase est précédée d'une phase de conditionnement pendant laquelle il n'y a pas de dialyse, mais uniquement une hémofiltration.

7. Dispositif selon l'une quelconque des revendications précédentes, **caractérisé en ce que** l'unité de commande est configurée de telle sorte que la durée de la première phase dépend d'un ou de plusieurs paramètres de traitement et/ou du patient.

8. Dispositif selon la revendication 7, **caractérisé en ce que** les paramètres du patient consiste en le poids corporel et/ou le volume de distribution du patient et/ou la concentration de substance dans le sang, comme p. ex. la concentration prédialytique d'urée.

9. Dispositif selon l'une quelconque des revendications précédentes, **caractérisé en ce que** l'unité de commande est conçue de telle sorte que, dans la deuxième phase, le débit et/ou la concentration sont ajustés en fonction de la clairance déterminée ou de la dose de dialyse obtenue pendant le traitement.

10. Dispositif selon l'une quelconque des revendications précédentes, **caractérisé en ce que** l'unité de commande est réalisée de telle sorte que le passage de la première à la deuxième phase a lieu en continu ou par étapes en ce qui concerne la concentration et/ou en ce qui concerne le débit de dialysat.

11. Dispositif selon l'une quelconque des revendications précédentes, **caractérisé en ce que** l'unité de commande est configurée pour faire fonctionner le dispositif en tant qu'appareil d'hémodialyse ou en tant qu'appareil d'hémodiafiltration et, le cas échéant, temporairement en tant qu'appareil d'hémofiltration.
